# EUROPÄISCHE PATENTSCHRIFT

(11) **EP 3 713 909 B1**
(45) Veröffentlichungstag und Bekanntmachung des Hinweises auf die Patenterteilung: **26.05.2021**
(21) Anmeldenummer: 17818428.9
(22) Anmeldetag: 21.11.2017
(51) Int. Cl.: C07C 67/055, C07C 69/15

(54) **VERFAHREN ZUR HERSTELLUNG VON VINYLACETAT**
METHOD FOR PRODUCING VINYL ACETATE
PROCÉDÉ DE PRÉPARATION D'ACÉTATE DE VINYLE

(43) Veröffentlichungstag der Anmeldung: 30.09.2020
(73) Patentinhaber: Wacker Chemie AG, 81737 München (DE)
(72) Erfinder: DAFINGER, Willibald, 94133 Röhrnbach (DE); PATSCH, Brigitte, 84489 Burghausen (DE); RUDOLF, Günther, 84453 Mühldorf (DE)
(74) Vertreter: Ege, Markus
(86) Internationale Anmeldenummer: PCT/EP2017/079933
(87) Internationale Veröffentlichungsnummer: WO 2019/101296

(56) Entgegenhaltungen:
- WO-A1-2009/130211
- WO-A1-2010/149527

## Beschreibung

Die Erfindung betrifft ein Verfahren zur Herstellung von Vinylacetat in einem heterogen katalysierten, kontinuierlichen Gasphasenprozess durch Umsetzung von Ethylen mit Essigsäure und Sauerstoff in einem Festbettröhrenreaktor und Aufarbeitung des Produktgasstromes, wobei ein überwiegend aus Ethylen, Kohlendioxid, Ethan, Stickstoff und Sauerstoff bestehendes Gasgemisch (= Kreisgas) im Kreis geführt wird, und das Kreisgas vor dem Festbettröhrenreaktor mit den Edukten Essigsäure, Ethylen und Sauerstoff versetzt wird und mittels mit Heizdampf betriebenen Wärmetauschern auf Reaktionstemperatur gebracht wird, und wobei die Anreicherung des Kreisgases mit Essigsäure in einem Essigsäuresättiger erfolgt.

Vinylacetat-Monomer (VAM) kann in einem kontinuierlichen Verfahren unter Rückführung des aufgereinigten Produktstromes hergestellt werden (Kreisgas-Prozess). Dabei reagiert in einer heterogen katalysierten Gasphasenreaktion Ethylen mit Essigsäure und Sauerstoff an Katalysatoren, welche im Allgemeinen Palladium- und Alkalimetallsalze auf einem Trägermaterial enthalten und zusätzlich noch mit Gold oder Rhodium dotiert sein können. Bevorzugt wird ein Pd/Au-Katalysatorgemisch mit Kaliumacetat-Promotor eingesetzt.

Die Edukte Ethylen, Sauerstoff und Essigsäure werden in einer exothermen Reaktion (VAM: _{B}H°₂₉₉ = - 176 kJ/Mol), im Allgemeinen bei einem Überdruck von 7 bis 15 bar und, je nach Laufzeit des Katalysators, bei einer Temperatur von im Allgemeinen von 130°C bis 200°C, in einem Festbettröhrenreaktor, aber auch Fluidbettreaktoren, zu Vinylacetat umgesetzt:

C₂H₄ + CH₃COOH + ½ O₂ -> CH₃COOCH=CH₂ + H₂O

Hauptnebenreaktion ist dabei die Ethylen-Totaloxidation zu CO₂: C₂H₄ + 3 O₂ -> 2 CO₂ + 2 H₂O (CO₂: _{B}H°₂₉₉ = - 1322 kJ/Mol) Der Ethylenumsatz liegt im Allgemeinen bei etwa 5 bis 20 %, der Essigsäureumsatz bei 20 bis 60 % und der Sauerstoffumsatz bei bis zu 90 %.

Wegen des unvollständigen Umsatzes von Ethylen wird bei der Herstellung von Vinylacetat ein überwiegend aus Ethylen, Kohlendioxid, Ethan, Stickstoff und Sauerstoff bestehendes Gasgemisch (= Kreisgas) im Kreis geführt. Das Kreisgas wird vor dem Festbettröhrenreaktor mit den Edukten Essigsäure, Ethylen und Sauerstoff versetzt und mittels mit Heizdampf betriebenen Wärmetauschern auf Reaktionstemperatur gebracht. Die Anreicherung des Kreisgases mit Essigsäure erfolgt üblicherweise mittels einem mit Heizdampf geheizten Essigsäuresättiger.

Die Beladung von Kreisgas mit Essigsäure im Essigsäuresättiger hat allerdings den Nachteil, dass es bereits nach kurzen Laufzeiten zur Verschmutzung des Essigsäuresättigers kommt. Bei dem Sättiger handelt es sich im Allgemeinen um eine Kolonne in welchem trockenes Kreisgas (ohne Essigsäure und Wasser) zunächst direkt in die Kolonne von unten nach oben geführt wird und Essigsäure dosiert zugeführt wird. Vor allem im unteren Kolonnenbereich kommt es an der Einleitstelle des trockenen und heißen Kreisgases zu Verschmutzungen, welche die Produktionskapazität beeinträchtigen und sogar eine Abstellung der Produktion zur Reinigung auslösen.

In der WO 2009/130211 A1 wird beschrieben, dass mittels einer Vorsättigung des Kreisgases mit Rückessigsäure in einem dem Essigsäuresättiger vorgeschalteten Vorsättiger die Verschmutzung im Essigsäuresättiger reduziert werden kann.

Die EP 1 655 279 B1 beschreibt ein Verfahren zur Sättigung des Kreisgases mit Essigsäure in einem Essigsäuresättiger, wobei die dem Essigsäuresättiger entnommene Flüssigkeit in zwei Teilströme aufgeteilt wird und ein Teilstrom in den Essigsäuresättiger unter Aufrechterhaltung eines Mindestumpumps zurückgeführt wird. Mit diesem Verfahren wird angeblich das Verschmutzungsproblem im Essigsäuresättiger umgangen.

Der Erfindung lag die Aufgabe zugrunde, ein Verfahren zur Verfügung zu stellen, mit welchem sich die Verschmutzung des Essigsäuresättigers ohne die genannten apparativen Mittel wirksam verhindern lasst.

Aus dem Stand der Technik war bekannt, dass sich bei der Aufarbeitung des Produktgasgemisches aus der Gasphasenoxidation die Verschmutzung der dabei eingesetzten Kolonnen mittels Zugabe von Verbindungen mit mindestens einer N-Oxyl-Radikal-Gruppe -N-O· verhindern lässt. Die KR 10-2008-97529 beschreibt die Zugabe von 2,2,6,6-Tetramethylpiperidinyloxyl (TEMPO) oder von 4-Oxo-TEMPO bei der Aufarbeitung des Kreisgases. Die WO 2010/149527 A1 beschreibt die Zugabe von TEMPO oder OH-TEMPO bei der Aufarbeitung des VAM-haltigen Produktgasstroms. In der WO 2015/082450 A1 wird dazu die N-Oxyl-Verbindung als wässrige Lösung eingesetzt. Die WO 2012/058196 A1 beschreibt die Zugabe von Radikalfängern (Scavenger) zur Behandlung des Produktgasstromes aus der Gasphasenoxidation von Ethylen und Essigsäure zu Vinylacetat.

Gegenstand der Erfindung ist ein Verfahren zur Herstellung von Vinylacetat in einem heterogen katalysierten, kontinuierlichen Gasphasenprozess durch Umsetzung von Ethylen mit Essigsäure und Sauerstoff in einem Festbettröhrenreaktor und Aufarbeitung des Produktgasstromes, wobei ein überwiegend aus Ethylen, Kohlendioxid, Ethan, Stickstoff und Sauerstoff bestehendes Gasgemisch (= Kreisgas) im Kreis geführt wird, und das Kreisgas vor dem Festbettröhrenreaktor mit den Edukten Essigsäure, Ethylen und Sauerstoff versetzt wird und mittels mit Heizdampf betriebenen Wärmetauschern auf Reaktionstemperatur gebracht wird, und wobei die Anreicherung des Kreisgases mit Essigsäure in einem Essigsäuresättiger erfolgt, dadurch gekennzeichnet, dass dem Essigsäuresättiger ein oder mehrere N-Oxyl-Verbindungen, welche mindestens eine N-Oxyl-Radikal-Gruppe -N-O- enthalten, aus der Gruppe enthaltend 2,2,6,6-Tetramethylpiperidinyloxyl, 4-Hydroxy-2,2,6,6-tetramethylpiperidinyloxyl, 4-Oxo-2,2,6,6-tetramethylpiperidinyloxyl und 4-Ethanoyloxy-2,2,6,6-tetramethylpiperidinyloxyl, in einer solchen Menge zugegeben wird, dass im Kolonnensumpf des Essigsäuresättigers die Konzentration der N-Oxyl-Verbindung 10 bis 100 Gewichts-ppm beträgt, bezogen auf das Gewicht des Kolonnensumpfes des Essigsäuresättigers.

Bevorzugt werden 2,2,6,6-Tetramethylpiperidinyloxyl (TEMPO) und 4-Hydroxy-2,2,6,6-tetramethylpiperidinyloxyl (4-OH-TEMPO).

Die N-Oxyl-Verbindung wird im Allgemeinen als Lösung dosiert. Geeignete Lösungsmittel sind Vinylacetat, Vinylacetat-Wasser-Mischungen oder Wasser. Vorzugsweise wird Vinylacetat oder eine Vinylacetat-Wasser-Mischung als Lösungsmittel eingesetzt. Besonders bevorzugt wird die N-Oxyl-Verbindung in Form einer 5 bis 10 Gew.-%-igen Lösung in einer Vinylacetat-Wasser-Mischung zugesetzt.

Die für das Verfahren erforderliche Menge an N-Oxyl-Verbindung hängt zum einen von der Konstruktion und Kapazität der jeweiligen Anlage ab. Im Allgemeinen werden 200 bis 500 Gewichts-ppm, vorzugsweise 200 bis 400 Gewichts-ppm an N-Oxyl-Verbindung, jeweils bezogen pro Tonne Vinylacetat im Produktgasstrom, zugeführt.

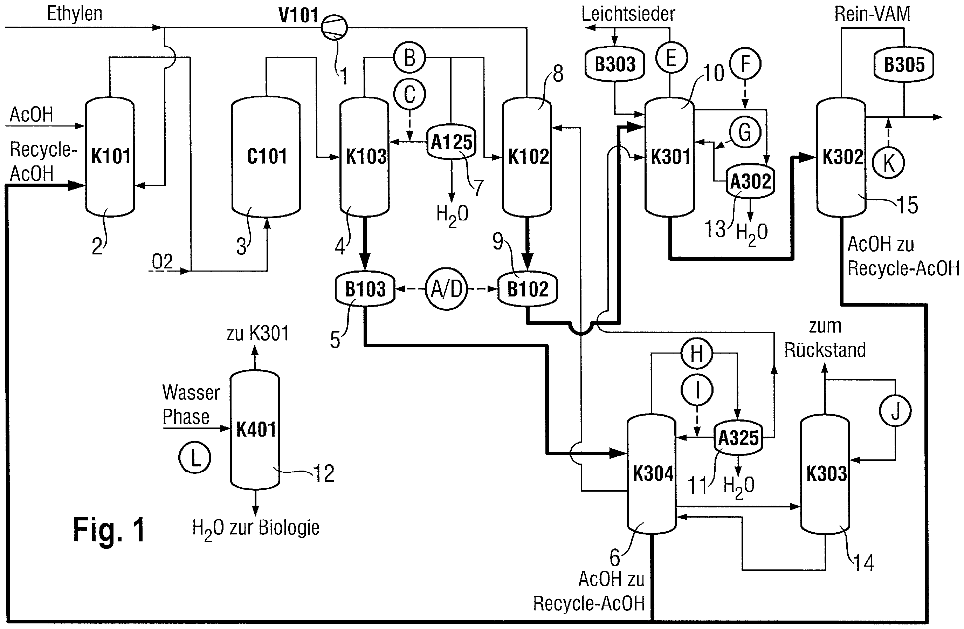

Die N-Oxyl-Verbindung kann dem Essigsäuresättiger K-101 (2) als Bestandteil der Frischessigsäure (Figur 1: AcOH) oder als Bestandteil der Recycle-Essigsäure (Figur 1: Recycle-AcOH) oder sowohl mit der Frischessigsäure als auch mit der Recycle-Essigsäure zugeführt werden.

Die N-Oxyl-Verbindung wird in einer solchen Menge zugegeben, dass im Kolonnensumpf des Essigsäuresättigers die Konzentration der N-

Oxyl-Verbindung 10 bis 100 Gewichts-ppm, vorzugsweise 10 bis 50 Gewichts-ppm, beträgt.

Besonders bevorzugt wird eine Ausführungsform bei der die N-Oxyl-Verbindung bei der Aufarbeitung des Produktgasstromes an einer oder mehreren Stellen zugegeben wird, welche in Figur 1 als Stellen A bis K benannt sind, und dort in einer solchen Menge zugegeben wird, dass sich, über die Zuführung als Bestandteil der Recycle-Essigsäure, im Kolonnensumpf des Essigsäuresättigers eine Konzentration der N-Oxyl-Verbindung von 10 bis 100 Gewichts-ppm, vorzugsweise 10 bis 50 Gewichts-ppm, einstellt.

In Figur 1 ist ein vereinfachtes Schema für die Herstellung von Vinylacetat in einem Gasphasenprozess und anschließender Aufarbeitung des Produktgasstromes angegeben.

Das mit dem Kreisgasverdichter V-101 (1) verdichtete Kreisgas wird mit Frischethylen, das den bei der Reaktion verbrauchten Ethylenanteil ersetzt, angereichert und dem Essigsäuresättiger K-101 (2) zugeführt. Die in der Reaktion umgesetzte Essigsäure wird im Essigsäuresättiger K-101 (2) durch Einspeisung von Frischessigsäure und Recycle-Essigsäure ersetzt. Die Hochsieder und andere Nebenprodukte, wie beispielsweise alle rückgeführten Polymere und nicht verbrauchte Inhibitoren werden am Sumpf des Essigsäuresättigers (2) abgezogen, von Rest-Essigsäure in der Essigsäureaufarbeitung befreit und die verbleibenden Rückstände entsorgt.

Da in der Reaktion keine vollständige Umsetzung der Essigsäure erfolgt, wird in den nachfolgenden Destillationen (beispielsweise in der Azeotrop-Kolonne K-304 (6) und der Rein-VAM-Kolonne K-302 (15)) diese Essigsäure jeweils am Sumpf abgezogen und dem Reycycle-Essigsäure-Tank zugeführt.

Dem den Essigsäuresättiger K-101 (2) verlassenden, mit Essigsäure beladenen Kreisgas wird vor Eintritt in das Reaktorsystem über eine Düse Sauerstoff zugegeben. Anschließend wird das Kreisgas mit einem Kreisgasdruck von 7 bis 15 bar abs. dem Festbettröhrenreaktor C-101 (3) zugeführt, welcher mit einem Pd/Au-Katalysatorgemisch mit Kaliumacetat-Promotor beladen ist, und bei einer Temperatur von 130 bis 200°C betrieben wird.

Der den Festbettröhrenreaktor C-101 (3) verlassende Gasstrom wird dem unteren Teil der Vorentwässerungs-Kolonne K-103 (4) zugeführt. Ein erstes Kondensat (Vinylacetat, Wasser und nicht umgesetzte Essigsäure) aus dieser Kolonne wird in den Roh-VAM-Behälter B-103 (5) geleitet. Der Roh-VAM-Behälter B-103 (5) ist Stelle A an der eine Zugabe der N-Oxyl-Verbindung erfolgen kann.

Das Roh-VAM aus dem Roh-VAM-Behälter B-103 (5) wird danach in die Azeotrop-Kolonne K-304 (6) gepumpt. Der Hauptgas-Strom aus der Vorentwässerungs-Kolonne K-103 (4) kann vor der nachfolgenden Kondensation im Kreisgaswäscher K-102 (8) mit der N-Oxyl-Verbindung versetzt werden (Stelle B). Zur Inhibierung des Rücklaufs aus dem Phasentrenner A-125 (7) kann die N-Oxyl-Verbindung auf dem Weg zur Vorentwässerungskolonne K-103 (4) zugegeben werden (Stelle C).

Der nicht kondensierte Bestandteil der Kopfbrüden der Vorentwässerungskolonne (4), im wesentlichen Ethylen, CO₂ und Vinylacetat wird zum Kreisgaswäscher K-102 (8) abgegeben. Die nicht kondensierten VAM-Anteile werden im mit Essigsäure betriebenen Kreisgaswäscher K-102 (8) absorbiert. Die zur Kreisgaswäsche erforderliche Absorptions-AcOH kann von der Azeotrop-Kolonne K-304 (6) zugeführt werden. Das nun VAM-freie Kreisgas wird über den Kreisgas-Verdichter V-101 (1) und den Essigsäuresättiger K-101 (2) wieder der Reaktion im Reaktor C-101 (3) zugeführt. Das Sumpfprodukt aus dem Kreisgaswäscher K-102 (8) wird in den Roh-VAM-Behälter B-102 (9) geleitet und von dort in die Entwässerungs-Kolonne K-301 (10). In den Roh-VAM-Behälter B-102 (9) und/oder in den ROH-VAM-Behälter B-103 (5) kann ebenfalls N-Oxyl-Verbindung zugegeben werden (Stelle D) .

Vorzugsweise wird in die Entwässerungs-Kolonne K-301 (10) im Allgemeinen noch ein zweiter Roh-VAM-Strom geleitet: Das Roh-VAM aus dem Roh-VAM-Behälter B-103 (5) (= Kondensat aus Vorentwässerung (4)). Dieses wird zunächst in der AzeotropKolonne K-304 (6) destilliert. Das Kopfprodukt dieser Destillation in der Azeotrop-Kolonne K-304 (6), im Wesentlichen Vinylacetat und Wasser, wird zur Wasser-Abtrennung in den Phasentrenner A-325 (11) überführt, und kann zwischen AzeotropKolonne (6) und Phasentrenner (11) mit der N-Oxyl-Verbindung versetzt werden (Stelle H).

Der Großteil der organischen Phase (im Wesentlichen VAM) aus dem Phasentrenner A-325 (11) wird als Rücklauf wieder in die Azeotrop-Kolonne K-304 (6) zurückgepumpt und kann vorher mit der N-Oxyl-Verbindung versetzt werden (Stelle I). Der verbleibende Teil der organischen Phase wird zur EntwässerungsKolonne K-301 (10) verbracht.

Die wässerige Phase des Phasentrenners A-325 (11) wird zur Abwasser-Kolonne K-401 (12) gefördert, in der alle wässerigen Phasen aus den Phasentrennern A-125 (7), Phasentrenner A-302 (13) und Phasentrenner A-325 (11) der gesamten Destillation aufgearbeitet werden. Das wässerige Sumpfprodukt der Abwasser-Kolonne (12) wird entsorgt, das Kopfprodukt in die Entwässerungskolonne K-301 (10) zurückgeführt.

Ein Seitenabzug der Azeotrop-Kolonne K-304 (6) kann zur Entfernung des Ethylacetats zur Ethylacetat-Kolonne K-303 (14) geleitet werden. Im Rücklauf dieser Ethylacetat-Kolonne K-303 (14) kann ebenfalls N-Oxyl-Verbindung zudosiert werden (Stelle J) .

Vorzugsweise wird in der Entwässerungskolonne K-301 (10) im Wesentlichen das Sumpfprodukt aus dem Kreisgaswäscher K-102 (8), welches im Wesentlichen Wasser, Vinylacetat, Essigsäure und Leichtsieder (vor allem Acetaldehyd) enthält, aufgetrennt: Die Leichtsieder und Wasser werden von Vinylacetat und Essigsäure abgetrennt. Dabei wird über den Kopf der Entwässerungskolonne K-301 (10) der hier durch Vinylacetat-Hydrolyse gebildete Acetaldehyd abgetrennt, nachfolgend kondensiert und zur weiteren Aufarbeitung gepumpt. An dieser Stelle kann ebenfalls N-Oxyl-Verbindung zugegeben werden (Stelle E).

Über einen Zwischenboden der Entwässerungskolonne K-301 (10) kann der wasserhaltige Seitenabzug zum Phasentrenner A-302 (13) geleitet werden. An dieser Leitung kann N-Oxyl-Verbindung zugegeben werden (Stelle F). Die organische Phase wird gegebenenfalls ebenfalls mit der N-Oxyl-Verbindung versetzt (Stelle G) und dann, an gegebenenfalls mehreren Stellen, als Rücklauf der Entwässerungskolonne K-301 (10) wieder zugeführt.

Das Sumpfprodukt der Entwässerungskolonne K-301 (10), Vinylacetat und Essigsäure, wird zur Rein-VAM-Kolonne K-302 (15) geleitet. Über Kopf wird Rein-VAM abgetrennt und teilweise als Rücklauf zurückgeführt. An diesem Rücklauf kann ebenfalls die N-Oxyl-Verbindung zugegeben werden (Stelle K). Das Sumpfprodukt, im Wesentlichen Essigsäure, wird der EssigsäureAufarbeitung zugeführt.

In einer besonders bevorzugten Ausführungsform wird die N-Oxyl-Verbindung mindestens einer der Kolonnen aus der Gruppe Azeotrop-Kolonne K-304 (6), Entwässerungskolonne K-301 (10), Rein-VAM-Kolonne K-302 (15) zugegeben. In Figur 1 ist der Weg der N-Oxyl-Verbindung zum Essigsäuresättiger bei Zugabe zu diesen Kolonnen mit einer fetten Linie eingezeichnet.

In diesem Schema in Figur 1 werden die CO₂-Wäsche und die Essigsäure-Aufarbeitung nicht beschrieben.

Mit der erfindungsgemäßen Vorgehensweise werden nicht nur in den Kolonnen zur Aufarbeitung des Produktgasstromes die Polymerbildungsraten und damit die Verschmutzung deutlich reduziert, sondern auch im Essigsäuresättiger-System das Fouling deutlich reduziert. Dies wird bei Zugabe zu den Kolonnen, über eine oder mehrere der Stellen A bis K, durch eine Überdosierung erreicht, das heißt es wird mehr N-Oxyl-Verbindung zugegeben, als zur Inhibierung des Foulings an diesen Stellen jeweils benötigt wird, und die überschüssige und nicht abreagierte N-Oxyl-Verbindung gelangt folglich über die Recycle-Essigsäure in das Essigsäuresättiger-System. Die Zeitintervalle zwischen den Reinigungen der Kolonnen oder den Filterwechseln werden verlängert, die Anlagenverfügbarkeit wird erhöht und damit die VAM-Ausbeute verbessert. Nicht zuletzt wird mit der wirksamen Reduzierung des Foulings in dem Essigsäuresättiger ein wichtiger Beitrag zur Erhöhung der Betriebssicherheit erhalten.

Die nachfolgenden Beispiele dienen der weiteren Erläuterung der Erfindung:

### Beispiel 1:

In einer Anlage gemäß Figur 1, welche unter den oben genannten Bedingungen (Kreisgasdruck 7 bis 15 bar abs., Reaktionstemperatur 130 bis 200°C) mit einer Gas Hourly Space Velocity (GHSV) von ca. 3000 bis 4000 [1/h] und einer Space Time Yield (STY) von 600 bis 1200 (kg VAM/ m³ Kat x h) betrieben wurde, wurde jeweils an den Stellen A bis L 200 bis 400 Gewichts-ppm 4-Hydroxy-2,2,6,6-tetramethylpiperidin-1-oxyl (4-OH-TEMPO), jeweils als Lösung in einer Vinylacetat-Wasser-Mischung, zugegeben, so dass im Sumpf des Essigsäuresättigers K-101 (2) das 4-OH-TEMPO in einer Menge von 10 bis 50 Gewichts-ppm vorgelegen hat.

Zur Beurteilung der Verschmutzung des Essigsäuresättigers K-101 (2) wurde über einen Zeitraum von 7 Monaten der Wärmedurchgangskoeffizient k in W/(m² K) bestimmt. Je größer die Verschmutzung (Fouling) am Essigsäuresättiger, desto geringer der Wärmeübergang am Essigsäuresättiger, und desto kleiner der Wert k.

Der Wärmedurchgangskoeffizient k betrug am Anfang der Messreihe 750 W/ (m² K) und nach sieben Monaten 650 W/(m² K) . Vergleichsbeispiel 2:
Es wurde analog Beispiel 1 vorgegangen, mit dem Unterschied, dass jeweils an den Stellen A bis L nur 100 bis 200 Gewichts-ppm 4-Hydroxy-2,2,6,6-tetramethylpiperidin-1-oxyl (4-OH-TEMPO), jeweils als Lösung in einer Vinylacetat-Wasser-Mischung, zugegeben wurden, und kein 4-OH-TEMPO in den Essigsäuresättiger K-101 (2) gelangte.

Der Wärmedurchgangskoeffizient k betrug am Anfang der Messreihe 750 W/ (m² K) und nach sieben Monaten 300 W/ (m² K) .

Während bei der erfindungsgemäßen Vorgehensweise (Beispiel 1) der k-Wert über eine Laufzeit von 7 Monaten nur um 13 % abgenommen hat, hat der k-Wert ohne die erfindungsgemäße Maßnahme (Vergleichsbeispiel 2) binnen 7 Monate 80 % an Wert verloren.

## Patentansprüche

1. Verfahren zur Herstellung von Vinylacetat in einem heterogen katalysierten, kontinuierlichen Gasphasenprozess durch Umsetzung von Ethylen mit Essigsäure und Sauerstoff in einem Festbettröhrenreaktor und Aufarbeitung des Produktgasstromes, wobei ein überwiegend aus Ethylen, Kohlendioxid, Ethan, Stickstoff und Sauerstoff bestehendes Gasgemisch (= Kreisgas) im Kreis geführt wird, und das Kreisgas vor dem Festbettröhrenreaktor mit den Edukten Essigsäure, Ethylen und Sauerstoff versetzt wird und mittels mit Heizdampf betriebenen Wärmetauschern auf Reaktionstemperatur gebracht wird, und wobei die Anreicherung des Kreisgases mit Essigsäure in einem Essigsauresättiger erfolgt, **dadurch gekennzeichnet, dass** dem Essigsäuresättiger ein oder mehrere N-Oxyl-Verbindungen, welche mindestens eine N-Oxyl-Radikal-Gruppe -N-O. enthalten, aus der Gruppe enthaltend 2,2,6,6-Tetramethylpiperidinyloxyl, 4-Hydroxy-2,2,6,6-tetramethylpiperidinyloxyl, 4-Oxo-2,2,6,6-tetramethylpiperidinyloxyl und 4-Ethanoyloxy-2,2,6,6-tetramethylpiperidinyloxyl, in einer solchen Menge zugegeben wird, dass im Kolonnensumpf des Essigsäuresättigers die Konzentration der N-Oxyl-Verbindung 10 bis 100 Gewichts-ppm beträgt, bezogen auf das Gewicht des Kolonnensumpfes des Essigsäuresättigers.

2. Verfahren nach Anspruch 1, **dadurch gekennzeichnet, dass** die N-Oxyl-Verbindung mit mindestens einem der beiden Ströme, Frischessigsäure und Recycle-Essigsäure, zugegeben wird.

3. Verfahren nach Anspruch 1 bis 2, **dadurch gekennzeichnet, dass** die N-Oxyl-Verbindung bei der Aufarbeitung des Produktgasstromes an einer oder mehreren Stellen zugegeben wird und in einer solchen Menge zugegeben wird, dass die N-Oxyl-Verbindung als Bestandteil der Recycle-Essigsäure dem Essigsäuresättiger zugeführt wird.

4. Verfahren nach Anspruch 3, **dadurch gekennzeichnet, dass** die N-Oxyl-Verbindung bei der Aufarbeitung des Produktgasstromes,
dem Kondensat aus der Vorentwässerungskolonne und/oder
dem Gasstrom aus der Vorentwässerungskolonne und/oder
dem Rücklauf zur Vorentwässerungskolonne und/oder
dem Sumpfprodukt aus dem Kreisgaswäscher und/oder
dem Kopfprodukt der Azeotropkolonne und/oder
dem Rücklauf in die Azeotropkolonne und/oder
dem Rücklauf in die Ethylacetat-Kolonne und/oder
dem Kopfprodukt der Entwässerungskolonne und/oder
dem Seitenabzug der Entwässerungskolonne und/oder
dem Rücklauf in die Entwäserungskolonne und/oder
dem Kopfprodukt der Reinvinylacetat-Kolonne
zugegeben wird.

5. Verfahren nach Anspruch 1 bis 4, **dadurch gekennzeichnet, dass** die N-Oxyl-Verbindung in einer solchen Menge zugegeben wird, dass im Kolonnensumpf des Essigsäuresättigers die Konzentration der N-Oxyl-Verbindung 10 bis 50 Gewichts-ppm, beträgt.

## Claims

1. Process for producing vinyl acetate in a heterogeneously catalyzed, continuous gas phase process via reaction of ethylene with acetic acid and oxygen in a fixed bed tubular reactor and workup of the product gas stream, wherein a gas mixture consisting predominantly of ethylene, carbon dioxide, ethane, nitrogen and oxygen (= cycle gas) is circulated, and the cycle gas is admixed with the reactants acetic acid, ethylene and oxygen upstream of the fixed bed tubular reactor and is brought to reaction temperature by means of heat exchangers operated with heating steam, and wherein the enrichment of the cycle gas with acetic acid is effected in an acetic acid saturator, **characterized in that** one or more N-oxyl compounds, containing at least one N-oxyl radical group -N-O·, from the group comprising 2,2,6,6-tetramethylpiperidinyloxyl, 4-hydroxy-2,2,6,6-tetramethylpiperidinyloxyl, 4-oxo-2,2,6,6-tetramethylpiperidinyloxyl and 4-ethanoyloxy-2,2,6,6-tetramethylpiperidinyloxyl are fed to the acetic acid saturator in such an amount that the concentration of the N-oxyl compound in the column bottoms of the acetic acid saturator is 10 to 100 ppm by weight, based on the weight of the column bottoms of the acetic acid saturator.

2. Process according to Claim 1, **characterized in that** the N-oxyl compound is added with at least one of the two streams, fresh acetic acid and recycle acetic acid.

3. Process according to Claim 1 to 2, **characterized in that** the N-oxyl compound is added at one or more points during the workup of the product gas stream and is added in such an amount that the N-oxyl compound is fed to the acetic acid saturator as a constituent of the recycle acetic acid.

4. Process according to Claim 3, **characterized in that** the N-oxyl compound during the workup of the product gas stream is added
to the condensate from the preliminary dewatering column and/or
to the gas stream from the preliminary dewatering column and/or
to the return stream to the preliminary dewatering column and/or
to the bottom product from the cycle gas scrubber and/or to the top product of the azeotrope column and/or
to the return stream into the azeotrope column and/or
to the return stream into the ethyl acetate column and/or
to the top product of the dewatering column and/or
to the side draw of the dewatering column and/or
to the return stream into the dewatering column and/or to the top product of the pure vinyl acetate column.

5. Process according to Claim 1 to 4, **characterized in that** the N-oxyl compound is added in such an amount that the concentration of the N-oxyl compound in the column bottoms of the acetic acid saturator is 10 to 50 ppm by weight.

## Revendications

1. Procédé pour la préparation d'acétate de vinyle dans un procédé en phase gazeuse en continu, catalysé de manière hétérogène par transformation d'éthylène avec de l'acide acétique et de l'oxygène dans un réacteur tubulaire à lit fixe et traitement du flux de gaz produit, un mélange de gaz (gaz de circulation) constitué majoritairement d'éthylène, de dioxyde de carbone, d'éthane, d'azote et d'oxygène étant mis en circulation, et le gaz de circulation étant mélangé avec les éduits acide acétique, éthylène et oxygène avant le réacteur tubulaire à lit fixe et étant amené à la température de réaction au moyen d'échangeurs de chaleur fonctionnant avec de la vapeur de chauffage, et l'enrichissement du gaz de circulation avec de l'acide acétique étant réalisé dans un saturateur à acide acétique, **caractérisé en ce qu'**un ou plusieurs composés de type N-oxyle, qui contiennent au moins un groupe radical N-oxyle -N-O·, du groupe contenant le 2,2,6,6-tétraméthylpipéridinyloxyle, le 4-hydroxy-2,2,6,6-tétraméthylpipéridinyloxyle, le 4-oxo-2,2,6,6-tétraméthylpipéridinyloxyle et le 4-éthanoyloxy-2,2,6,6-tétraméthylpipéridinyloxyle, est ajouté au saturateur à acide acétique en une telle quantité que la concentration du composé N-oxyle dans le fond de colonne du saturateur à acide acétique soit de 10 à 100 ppm en poids, par rapport au poids du fonds de colonne du saturateur à acide acétique.

2. Procédé selon la revendication 1, **caractérisé en ce que** le composé N-oxyle est ajouté avec au moins l'un des deux flux d'acide acétique frais et d'acide acétique recyclé.

3. Procédé selon les revendications 1 à 2, **caractérisé en ce que** le composé N-oxyle est ajouté en un ou plusieurs endroits lors du traitement du flux de gaz produit et en une telle quantité que le composé N-oxyle est alimenté au saturateur à acide acétique en tant qu'ingrédient de l'acide acétique recyclé.

4. Procédé selon la revendication 3, **caractérisé en ce que** le composé N-oxyle est ajouté, lors du traitement du flux de gaz produit,
au condensat provenant de la colonne de pré-déshydratation et/ou
au flux de gaz provenant de la colonne de pré-déshydratation et/ou
au reflux à la colonne de pré-déshydratation et/ou au produit de fond provenant de l'épurateur de gaz et/ou
au produit de tête de la colonne à azéotrope et/ou
au reflux dans la colonne à azéotrope et/ou
au reflux dans la colonne à acétate d'éthyle et/ou au produit de tête de la colonne de déshydratation et/ou
au soutirage latéral de la colonne de déshydratation et/ou
au reflux dans la colonne de déshydratation et/ou au produit de tête de la colonne à acétate de vinyle pur.

5. Procédé selon les revendications 1 à 4, **caractérisé en ce que** le composé N-oxyle est ajouté en une telle quantité que dans le fond de colonne du saturateur à acide acétique, la concentration du composé N-oxyle soit de 10 à 50 ppm en poids.
